# DEMANDE DE BREVET EUROPEEN

(11) **EP 0 653 216 A1**
(43) Date de publication de la demande: **17.05.1995**
(21) Numéro de dépôt: 94870177.6
(22) Date de dépôt: 16.11.1994
(51) Int. Cl.: A61M 3/02, A61M 5/44

(54) **Dispositif pour injecter une solution dans le corps d'un organisme**

(30) Priorité: 17.11.1993 BE 9301266
(71) Demandeur: CEDETEC S.A., L-9905 Troisvierges (LU)
(72) Inventeur: Charlier, André, B-4910 Theux (BE)
(74) Mandataire: Powis de Tenbossche, Roland

(57) **Abrégé**

Dispositif pour injecter une solution dans le corps d'un organisme à des fins thérapeutiques et/ou diagnostiques, ledit dispositif comprenant :
* une deuxième amenée (1) d'une solution sur laquelle est montée une vanne (2) ;
* une première amenée (3) d'une solution sur laquelle est montée une vanne (4) ;
* un système de chauffage (5) de solution provenant de la deuxième amenée (1) et/ou première amenée (3), et
* un éjecteur ou sortie (6)

ledit système de chauffage comprenant :
* une chambre (7) dans laquelle s'étend une résistance électrique (8) non isolée de la solution à chauffer, ladite résistance (8) étant associée à un organe de commande (9) de l'énergie électrique amenée à la résistance (8) ,et
* un système de régulation de l'énergie électrique amenée à ladite résistance (8) recevant un signal d'un capteur de température (11) de la solution sortant de la chambre (7) de chauffage et un signal d'un moyen (12,13) définissant la température désirée et émettant un signal de commande vers l'organe de commande (9);
   au moins une (2) desdites vannes émettant un signal vers un moyen (17) destiné à corriger le signal émis par le système de régulation (10).

## Description

La présente invention a pour objet un dispositif pour injecter une solution dans le corps d'un organisme à des fins thérapeutiques et/ou diagnostiques.

Ce dispositif comprend :
* une première amenée d'une solution sur laquelle est montée une vanne ;
* une deuxième amenée d'une solution sur laquelle est montée une vanne ;
* un système de chauffage de solution provenant de la première amenée et/ou deuxième amenée, et
* un éjecteur ou sortie.

Le dispositif suivant l'invention est destiné à obtenir en un temps très court la température désirée avec une grande précision, par exemple à obtenir en moins d'environ 1 seconde une température se différenciant de la température désirée de moins de 1° C, en particulier d'environ 0,5° C.

Dans le dispositif suivant l'invention, le système de chauffage comprend :
* une chambre dans laquelle s'étend une résistance électrique non isolée de la solution à chauffer, ladite résistance étant associée à un organe de commande de l'énergie électrique amenée à la résistance ;
* un système de régulation de l'énergie électrique amenée à ladite résistance recevant un signal d'un capteur de température de la solution sortant de la chambre de chauffage et un signal émanant de la valeur de consigne définissant la température désirée, et émettant un signal de commande vers l'organe de commande.

Pour obtenir rapidement une température proche de la température désirée, au moins une desdites vannes émet un signal vers un moyen destiné à corriger le signal émis par le système de régulation.

Un tel dispositif convient pour le lavage du colon ou autres parties de l'intestin, mais convient également en faisant varier la température, à permettre à un médecin ou vétérinaire de donner un diagnostic et d'appliquer une thérapeutique en fonction des réponses de l'organisme.

Dans une forme de réalisation, la chambre est agencée de manière à ce que le temps de séjour moyen de solution provenant d'une amenée dans la chambre est inférieur à 3 secondes, de préférence à une seconde.

De façon avantageuse, la chambre est agencée de manière à ce que la vitesse moyenne de la solution provenant d'une amenée dans la chambre est supérieure à 1,5 m/s, de préférence supérieure à 2,5 m/s ; ceci permettant un excellent échange ou transfert de chaleur entre la résistance et la solution.

De manière à pouvoir réaliser des lavages et/ou diagnostics à des débits différents, mais débits correspondant aux débits désirés, la première amenée et sa vanne sont agencées de manière à permettre un débit de solution compris entre un débit minimal et un débit maximal.

De façon avantageuse le débit maximal de solution provenant de la deuxième amenée est supérieur à au moins deux fois le débit maximal de la première amenée.

Dans des formes de réalisation, la vanne de la deuxième amenée est une vanne tout ou rien, ladite vanne émettant un signal vers un moyen destiné à corriger le signal émis par le système de régulation, le débit maximum passant par ladite vanne étant de préférence inférieur à 100 l/h, et/ou la première amenée est de préférence munie d'une première vanne tout ou rien, et d'une deuxième vanne réglant le débit de solution vers la chambre de chauffage entre 20 et 50 l/h maximum, l'une desdites vannes, de préférence la première vanne émettant un signal vers un moyen destiné à corriger le signal émis par le système de régulation.

Dans le dispositif selon l'invention, le système de régulation comprend avantageusement un régulateur PI ou PD, de préférence PID (P : proportionnel, I : intégral, D : dérivé).

Le moyen définissant la température désirée est par exemple un bouton de réglage mais est avantageusement un programmateur permettant de définir une courbe de température désirée, par exemple une courbe continue (sinusoïdale, etc) ou présentant des sauts de température (par exemple, variation de température par paliers).

Pour assurer que la solution prenne la température désirée en un temps très court, la puissance maximale de la résistance est de préférence au moins 50 % supérieure à la puissance normalement nécessaire pour obtenir la température désirée pour le débit maximal et est avantageusement supérieur à deux fois la puissance normalement nécessaire pour que la température de la solution au débit minimal du dispositif corresponde à la température désirée.

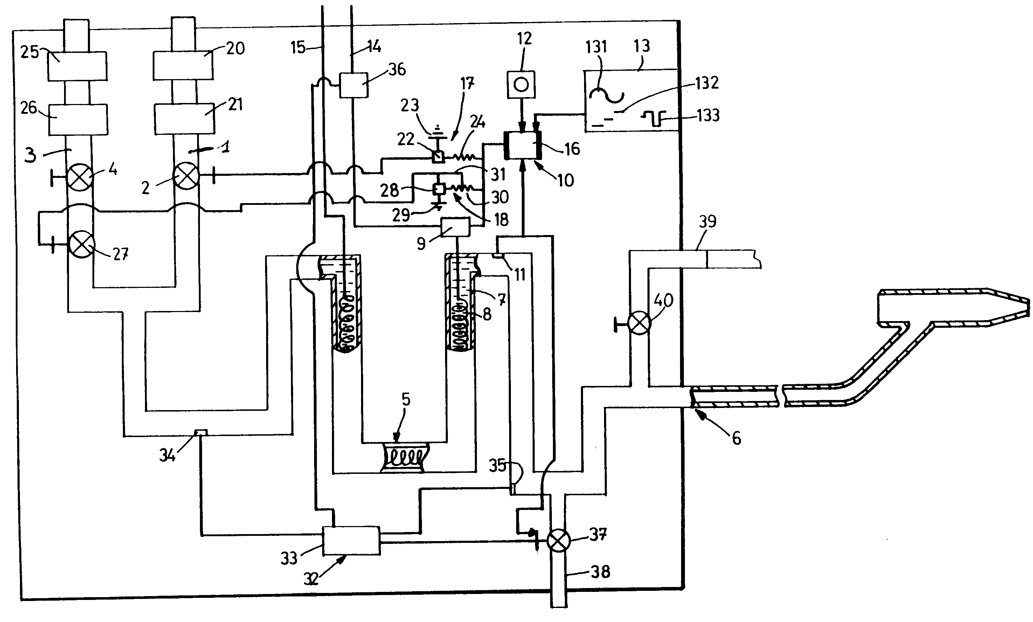

D'autres particularités et détails de l'invention ressortiront de la description détaillée suivante dans laquelle il est fait référence à la figure unique ci-annexée qui représente schématiquement une forme de réalisation d'un dispositif suivant l'invention.

Le dispositif représenté à la figure unique comprend :
* une deuxième amenée 1 d'une solution sur laquelle est montée une vanne 2 ;
* une première amenée 3 d'une solution sur laquelle est montée une vanne 4 ;
* un système de chauffage 5 de solution provenant de la première amenée 1 et/ou deuxième amenée 2 , et
* un éjecteur ou sortie 6.

Le système de chauffage 5 comprend :
* une chambre 7 dans laquelle s'étend une résistance électrique 8 non isolée de la solution à chauffer, ladite résistance 8 étant associée à un organe de commande 9 de l'énergie électrique amenée à la résistance 8 ;
* un système de régulation 10 de l'énergie électrique amenée à ladite résistance 8 recevant une signal d'un capteur de température 11 de la solution sortant de la chambre de chauffage 7 et un signal d'un moyen 12,13 définissant la température désirée et émettant un signal de commande vers l'organe de commande 9.

L'énergie électrique est amenée directement au dispositif via le réseau 220 V et via des fils électriques 14,15.

Toutefois, le dispositif est avantageusement muni d'un moyen pour assurer une tension sensiblement constante du courant amené à la résistance 8.

Le système de régulation 10 comprend un régulateur PID 16 recevant un signal d'un bouton de commande 12 pour fixer la température désirée (par exemple une température comprise entre 20 et 40°C, de préférence voisine de 37° C lorsque le dispositif est utilisé pour injecter un fluide ou solution dans le corps humain) ou un signal provenant d'un programmateur 13 pour définir par exemple une courbe continue (sinusoïdale) de température désirée 131, une courbe de température en palier 13 ou de forme carrée 133.

Le système de régulation 10 est associé à au moins un moyen (17 ou 18) pour corriger le signal électrique 19 émis par le régulateur 16, ce signal se présentant sous la forme d'un courant électrique.

Un tel moyen 18 par exemple un diviseur qui n'est actionné que pour de petits débits, c'est-à-dire qui n'est actionné dans le cas représenté que pour des débits compris entre 20 et 50 l/h. Par exemple, la première amenée 3 qui comprend avantageusement un filtre 25, un régulateur de pression 26 (2 bar ; 2 10⁵ Pa), une vanne tout ou rien 4 permettant le passage au maximum de 50 l/h de solution, comprend de préférence en outre une vanne réglant le débit sortant de ladite amenée entre 20 et 50 l/h.

Une desdites vannes 4 ou 27 de l'amenée 3, de préférence la vanne 27 commande un interrupteur 28 destiné à actionner le moyen 18 ou diviseur lorsque de la solution s'écoule dans ladite amenée 3. Un tel moyen est par exemple une résistance électrique 30 par laquelle un courant passe lorsque le débit est inférieur à 50 l/h.

Bien que la valeur de la correction (par exemple facteur de division voisin de 2 ou compris entre 2 et 10) ou du diviseur peut être une constante, cette valeur est de préférence fonction du débit passant dans la première amenée.

Dans ce cas préféré, un moyen 31 modifie la valeur de la résistance 30 dès que le débit est inférieur à 50 l/h (par exemple résistance maximale pour le débit maximal de la première amenée 3, à savoir 50 l/h dans le cas présent et résistance minimale pour le débit minimal de la première amenée 3, à savoir 20 l/h dans le cas présent).

Lorsqu'aucun débit de solution n'existe dans la première amenée 3, l'interrupteur 28 est en position ouverte empêchant tout passage de courant ou correction du signal électrique par le moyen 18.

Dans la forme de réalisation représentée, qui est une forme de réalisation préférée, un moyen de correction ou diviseur 17 est commandé par une vanne 2 montée sur la deuxième amenée. Dans cette forme de réalisation la deuxième amenée 1 comprend un filtre 20, un régulateur de pression (2 bar; 2 10⁵ Pa) 21 et une vanne 2 tout ou rien permettant le passage de 0 l/h de solution ou de 100 l/h de solution. Cette vanne 2 en position fermée commande un interrupteur 22 de manière à permettre le passage d'un courant par la branche 23 reliée à une terre via une résistance 24, c'est-à-dire de manière à diminuer l'intensité du signal électrique transmis à l'organe de commande lorsque le débit de solution à travers la chambre 7 n'est pas le débit maximum (100 l/h).

Le dispositif est avantageusement muni d'un système de sécurité 32 pour éviter toute surchauffe de la solution dans la chambre 7 en cas de surchauffe de la résistance ou d'un débit de solution anormal. Ce système comprend un moyen de mesure du débit 33, par exemple par différence de pression entre l'entrée et la sortie de la chambre 7 (capteurs 34,35), ce moyen 33 commandant un interrupteur 36 coupant l'alimentation du courant dès qu'une différence de pression trop faible est mesurée ou dès qu'une pression inférieure à une valeur prédéterminée est mesurée à l'entrée de la chambre par le capteur 34.

Le moyen 33, dès qu'une pression supérieure à une valeur est mesurée à la sortie de la chambre 7, commande une vanne 37 de manière à l'ouvrir et permettre le passage de la solution (par exemple de l'eau) dans le conduit 38 pour l'amener à un égout ou une décharge. Avantageusement cette vanne est également commandée par le capteur de température 11, de manière à évacuer la solution vers l'égout dès qu'une température supérieure à une température prédéterminée est mesurée.

La chambre 7 est agencée de manière à ce que la vitesse moyenne de solution dans la chambre, que le débit soit compris de 20 à 50 l/h ou de 100 l/h, soit d'environ 3 m/s et que le temps de séjour de la solution dans la chambre 7 soit de 0,5 - 0,8 seconde.

La chambre 7 avait une longueur de 12 cm et présentait une section transversale sensiblement circulaire, dont le diamètre était de 0,5 cm environ.

La résistance chauffante avait une puissance de 3000 W, c'est-à-dire si la température de solution à l'amenée 1 ou 3 est de l'ordre de 17° C et que la température de la solution à la sortie est de 37°C, une puissance supérieure à la puissance requise, en particulier lorsque le débit est compris entre 20 et 40 l/h.

Si nécessaire, des additifs, médicaments ou autres agents, etc peuvent être ajoutés à la solution avant la résistance chauffante ou après la résistance chauffante (conduit 39, vanne 40).

L'utilisation du dispositif décrit ci-avant a montré qu'il était possible d'obtenir une température de sortie de la solution correspondant sensiblement à la température désirée (^{T}sol - ^{T}désirée < 1°C en particulier 0,5°C) permettant ainsi à un médecin ou vétérinaire d'adapter en fonction du patient ou animal à traiter, la température de la solution, solution aqueuse ou eau de traitement par exemple pour le lavage du colon, etc.

De plus, en faisant varier la température de la solution -température qui est déterminée avec précision-, le médecin ou vétérinaire peut utiliser le dispositif pour donner un diagnostic en fonction des réponses ou sensations du patient ou animal traité (type d'obturation du colon, etc).

## Revendications

1. Dispositif pour injecter une solution dans le corps d'un organisme à des fins thérapeutiques et/ou diagnostiques, ledit dispositif comprenant :
* une deuxième amenée (1) d'une solution sur laquelle est montée une vanne (2) ;
* une première amenée (3) d'une solution sur laquelle est montée une vanne (4) ;
* un système de chauffage (5) de solution provenant de la première amenée (1) et/ou deuxième amenée (3), et
* un éjecteur ou sortie (6)
ledit système de chauffage comprenant :
* une chambre (7) dans laquelle s'étend une résistance électrique (8) non isolée de la solution à chauffer, ladite résistance (8) étant associée à un organe de commande (9) de l'énergie électrique amenée à la résistance (8) ,et
* un système de régulation (10) de l'énergie électrique amenée à ladite résistance (8) recevant un signal d'un capteur de température (11) de la solution sortant de la chambre (7) de chauffage et un signal d'un moyen (12,13) définissant la température désirée, et émettant un signal de commande vers l'organe de commande (9);
au moins une (2,4) desdites vannes émettant un signal vers un moyen (17,18) destiné à corriger le signal émis par le système de régulation (10).

2. Dispositif suivant la revendication 1, caractérisé en ce que la chambre (7) est agencée de manière à ce que le temps de séjour moyen de solution provenant d'une amenée (1,3) dans la chambre (7) est inférieur à 3 secondes, de préférence à une seconde.

3. Dispositif suivant la revendication 1 ou 2, caractérisé en ce que la chambre (7) est agencée de manière à ce que la vitesse moyenne de la solution provenant d'une amenée (1,3) dans la chambre (7) est supérieure à 1,5 m/s, de préférence supérieure à 2,5 m/s.

4. Dispositif suivant l'une des revendications 1 à 3, caractérisé en ce que la deuxième amenée (1) et sa vanne (2) sont agencées de manière à permettre un débit de solution supérieur au débit maximal de solution provenant de la première amenée (3) à ladite chambre (7) de chauffage.

5. Dispositif suivant la revendication 4, caractérisé en ce que la deuxième amenée (1) et sa vanne (2) sont agencées de manière à permettre un débit de solution compris entre un débit minimal et un débit maximal, ledit débit maximal étant supérieur à au moins deux fois le débit maximal de solution provenant de la première amenée (3) à ladite chambre (7) de chauffage.

6. Dispositif suivant l'une des revendications 1 à 5, caractérisé en ce que la vanne (2) de la première amenée (1) est une vanne tout ou rien, ladite vanne émettant un signal vers un moyen (17) destiné à corriger le signal émis par le système de régulation (10), le débit maximum passant par ladite vanne (2) étant de préférence inférieur à 100 l/h.

7. Dispositif suivant l'une des revendications 1 à 6, caractérisé en ce que la première amenée (3) est munie d'une première vanne tout ou rien (4), et d'une deuxième vanne (27) réglant le débit de solution vers la chambre de chauffage (7) entre 20 et 50 l/h , l'une (27) desdites vannes, de préférence la deuxième vanne émettant un signal vers un moyen (17) destiné à corriger le signal émis par le système de régulation (10).

8. Dispositif suivant l'une des revendications 1 à 7, caractérisé en ce que le système de régulation (10) comprend un régulateur proportionnel, intégral et dérivé (16).

9. Dispositif suivant l'une des revendications 1 à 8, caractérisé en ce que le moyen définissant la température désirée comprend un programmateur (13), ledit programmateur définissant de préférence une courbe continue ou non de la température désirée.

10. Dispositif suivant l'une des revendications 1 à 9, caractérisé en ce que la puissance maximale de la résistance (8) est au moins 50 % supérieure à la puissance normalement nécessaire pour obtenir la température désirée.
